# EUROPÄISCHE PATENTSCHRIFT

(11) **EP 1 874 394 B1**
(45) Veröffentlichungstag und Bekanntmachung des Hinweises auf die Patenterteilung: **07.01.2009**
(21) Anmeldenummer: 06724616.5
(22) Anmeldetag: 27.04.2006
(51) Int. Cl.: A61M 39/22

(54) **LEITUNG, INSBESONDERE LEITUNG EINES BLUTSCHLAUCH- ODER BLUTBEUTELSYSTEMS**
CONDUIT, ESPECIALLY OF A BLOOD TUBE SYSTEM OR BLOOD BAG SYSTEM
CONDUITE, EN PARTICULIER CONDUITE POUR SYSTEME DE TUBE DE PRELEVEMENT SANGUIN OU DE POCHE DE SANG

(30) Priorität: 28.04.2005 DE 102005019855
(43) Veröffentlichungstag der Anmeldung: 09.01.2008
(73) Patentinhaber: Fresenius HemoCare Deutschland GmbH, 61352 Bad Homburg (DE)
(72) Erfinder: MEISBERGER, Artur, 66606 St. Wendel (DE); HUBER, Alexandra, 63303 Dreieich (DE)
(74) Vertreter: Herrmann, Uwe
(86) Internationale Anmeldenummer: PCT/EP2006/003937
(87) Internationale Veröffentlichungsnummer: WO 2006/114319

(56) Entgegenhaltungen:
- DE-U1- 8 701 154
- FR-A- 1 129 284
- US-A- 4 913 401
- US-A- 5 071 411
- US-A- 6 132 413

## Beschreibung

Die Erfindung betrifft eine Leitung, insbesondere eine Leitung eines Blutschlauch- oder Blutbeutelsystems mit einem den Leitungsdurchlaß absperrenden Verschlusselement.

Bei heute verwendeten Blutschlauch- und Blutbeutelsystemen ist es bekannt, einen von Außen zu öffnenden, steril bleibenden Verschluß vorzusehen, der durch Brechen eines Kunststoffteils geöffnet wird. Nach dem Brechen des sogenannten Brechkonus wird ein Durchgang für die zuvor eingeschlossene bzw. abgesperrte Flüssigkeit freigegeben. Aus der US 6,132,413 ist ein Blutbeutel mit einer Leitung bekannt, die durch ein derartiges brechbares Element verschlossen ist. Zum Öffnen des Leitungsdurchgangs wird die Leitung mit dem darin befindlichen Element gebogen, was dazu führt, dass das Element bricht und dabei den Leitungsdurchgang freigibt. Ein Nachteil des aus dem Stand der Technik bekannten Verschlusses besteht darin, dass nur ein vergleichsweise kleiner Querschnitt für das durchströmende Medium freigegeben wird. Hinzu kommt, dass durch das Abbrechen von Kunststoffteilen ggf. Partikel entstehen, die in den Organismus eines Patienten gelangen können. Weiterhin nachteilig ist, dass scharfe Grate am Kunststoff auftreten, die beim Durchfluß von Blut Hämolyse erzeugen können. Als weiterer Nachteil ist schließlich zu nennen, dass das manuelle Brechen der Konen dem Personal bei häufiger Wiederholung dieser Tätigkeit Schmerzen bereiten kann.

Es ist die Aufgabe der Erfindung, eine Leitung der eingangs genannten Art dahingehend weiterzubilden, dass deren Verschlußelement derart geöffnet werden kann, dass ein vergleichsweise großer Querschnitt für das durchströmende Medium freigegeben wird und dass ferner die aus dem Stand der Technik bekannten Nachteile der Entstehung von Partikeln und scharfen Graten nicht auftreten. Des weiteren soll ermöglicht werden, dass das Verschlußelement auch maschinell geöffnet werden kann.

Diese Aufgabe wird durch eine Leitung mit den Merkmalen des Anspruchs 1 gelöst. Danach ist vorgesehen, dass in der Leitung ein plastisch verformbarer Verschlusskörper angeordnet ist, der in wenigstens einem Zustand die Leitung verschließt, und dass wenigstens der Abschnitt der Leitung, in dem sich der Verschlusskörper befindet, flexibel ausgeführt ist.

Der Begriff "Leitung" ist weit zu verstehen und umfaßt jeden beliebigen durchströmbaren Bereich, wie beispielsweise Leitungsabschnitte, Schläuche sowie Konnektorbereiche z. B. von Blutbeutelsystemen.

Im nicht verformten Zustand sperrt der Verschlusskörper die Leitung derart ab, dass der Durchgang für Medien versperrt ist. Zum Öffnen des Durchgangs wird der Verschlusskörper von Außen so verformt, dass ein Durchfluß von Medium möglich ist. Dabei muß die den Verschlusskörper umgebende Leitung derart flexibel sein, dass die Verformung des Verschlusskörpers möglich ist und anschließend ein Bereich für den Durchfluß des Mediums freigegeben wird. Dies kann durch Rückstellkräfte der Leitung selbst oder durch äußere Unterstützung erfolgen.

Der Verschlusskörper kann massiv, porös oder als Hohlkörper ausgeführt sein, der ein- oder mehrseitig offen oder der geschlossen ausgeführt ist. Wird der plastisch verformbare Verschlusskörper als Hohlkörper ausgeführt, ist das verbleibende Volumen des Verschlusskörpers nach dessen Deformation so gering, dass ein im wesentlichen ungehinderter Durchgang für das Medium gewährleistet ist.

Der Verschlusskörper läßt sich erfindungsgemäß derart ausführen, dass nach dessen Verformung keine scharfen Grate entstehen, die Blutschädigungen verursachen können, und während der Verformung auch keine Partikel entstehen, die in das Blut des Patienten gelangen könnten.

Da zum Öffnen des Verschlusses beispielsweise nur eine einfache Quetschbewegung notwendig ist, eignet sich die vorliegende Erfindung besonders gut für die maschinelle Verarbeitung bzw. Öffnung der Leitung. Denkbar ist selbstverständlich ebenfalls ein manuelles Öffnen, das vom Anwender bequem und ergonomisch möglich ist. ,

Das Wesen der Erfindung besteht darin, dass durch Krafteinwirkung der Verschlusskörper von einer Form, in der er die Leitung verschließt, in eine Form gebracht wird, die den Durchlaß durch die Leitung dauerhaft freigibt.

Der Verschlusskörper kann massiv sein, er kann hohl, offen oder geschlossen sein, er kann aus porösem Material bestehen oder auch hohl sein und eine Füllung aufweisen.

In einer denkbaren Ausführungsform ist der Verschlusskörper mit einer Flüssigkeit, vorzugsweise mit einer für Patienten verträglichen Flüssigkeit, wie z.B. mit einer Kochsalzlösung gefüllt. Dabei kann vorgesehen sein, dass die Flüssigkeit in einer Umhüllung, beispielsweise in einem Beutel aufgenommen ist, die bei Druckaufbringung beispielsweise mit der Hand zerplatzt und die Flüssigkeit freisetzt.

Um eine gute Haftung zwischen Verschlusskörper einerseits und der flexiblen Leitung andererseits zu gewährleisten, kann vorgesehen sein, dass der Verschlusskörper sowie der Abschnitt der Leitung, in dem sich der Verschlusskörper befindet, aus Materialien bestehen oder Materialien aufweisen, die aneinander haften. Dadurch kann gewährleistet werden, dass die Leitung bis zu einem vergleichsweise hohen Innendruck dicht bleibt.

Zusätzliche Möglichkeiten der Erhöhung des tolerierbaren Innendrucks bestehen in der Ausführung der Oberflächenbeschaffenheit des Verschlusskörpers und der Innenseite der Wandung der flexiblen Leitung, die beispielsweise porös bzw. rauh ausgeführt sein können, wodurch sich die Anhaftung des Verschlusskörpers an die Leitung verbessert.

Die Haftung zwischen Verschlusskörper und Leitung kann zusätzlich oder alternativ durch Materialien verbessert werden, die den Verschlusskörper und die Leitung miteinander verbinden, wie beispielsweise Klebstoff.

In weiterer Ausgestaltung der Erfindung ist vorgesehen, dass die Leitung einen ersten und einen zweiten Leitungsteil aufweist, die an wenigstens einer Verbindungsstelle fluiddicht miteinander in Verbindung stehen, wobei der Verschlusskörper in wenigstens einem Zustand den zweiten Leitungsteil verschließt und wobei der erste und der zweite Leitungsteil wenigstens abschnittsweise in dem Bereich des zweiten Leitungsteils, in dem sich der Verschlusskörper befindet, derart angeordnet sind, dass der erste Leitungsteil den zweiten Leitungsteil umgibt und beide Leitungsteile in diesem Bereich voneinander beabstandet oder voneinander lösbar sind.

Eine derartige Ausgestaltung der Leitung ist insbesondere dann von Vorteil, wenn hohe Anforderungen an die Druckfestigkeit des Verschlusses der Leitung gestellt werden. Denkbar ist beispielsweise, dass zwischen erster und zweiter Leitung, die beide flexibel ausgeführt sein können, ein ringförmiger, umlaufender Raum besteht oder bei Druckbeaufschlagung entsteht, in dem bei Druckbeaufschlagung der ersten Leitung ein entsprechender Druck herrscht. Dieser führt dazu, dass die zweite, innen liegende Leitung auf den Verschlusskörper gepreßt wird. Dadurch wird ein Öffnen des Verschlusses unabhängig vom Innendruck vollständig unterbunden.

Durch die Positionierung des Verschlusskörpers im Bezug auf die umlaufende Verbindung des ersten und zweiten Leitungsteils kann festgelegt werden, ob die Druckfestigkeit von einer oder von beiden Seiten des Verschlusskörpers gegeben ist.

Die zumindest im Bereich des Verschlusskörpers flexiblen Leitungen können auch Bestandteil eines Spritzgußteils oder nach einem anderen Verfahren hergestellte Leitungen sein.

Besonders bevorzugt ist es, wenn der erste und der zweite Leitungsteil zueinander konzentrisch angeordnet sind.

In weiterer Ausgestaltung der Erfindung ist vorgesehen, dass in dem Abschnitt, in dem der Verschlusskörper in dem zweiten Leitungsteil angeordnet ist, beide Leitungsteile voneinander beabstandet sind, und dass in einem hierzu benachbarten Bereich beide Leitungsteile mittels eines Verbindungsabschnittes, der beispielsweise konisch verläuft, miteinander verbunden sind.

In weiterer Ausgestaltung der Erfindung ist vorgesehen, dass der zweite Leitungsteil in dem ersten Leitungsteil aufgenommen ist und dass die Verbindungsstelle in dem Randbereich des zweiten Leitungsteils oder davon beabstandet, beispielsweise in einem zentralen Bereich des zweiten Leitungsteils angeordnet ist. Der Verschlusskörper kann ebenfalls mittig in dem zweiten Leitungsteil aufgenommen sein.

Der mittlere Bereich des Verschlusskörpers kann auf der Höhe der Verbindungsstelle liegen.

Weiterhin kann vorgesehen sein, dass der zweite Leitungsteil teilweise in dem ersten Leitungsteil angeordnet ist und teilweise aus diesem herausragt, wobei die Verbindungsstelle im Endbereich des ersten Leitungsteils angeordnet ist.

In weiterer Ausgestaltung der Erfindung ist ein Adapter vorgesehen, der auf den ersten und zweiten Leitungsteil aufgesteckt ist. Denkbar ist, dass die Leitungsteile unabhängig von dem Adapter eine Verbindungsstelle aufweisen und der Adapter z. B. auf den Endabschnitt der Leitungsteile aufgeklebt ist. Denkbar ist ferner, dass der Adapter die Leitungsteile zur Bildung der Verbindungsstelle fluiddicht miteinander verpresst. In einer möglichen Ausführungsform ist vorgesehen, dass der erste und zweite Leitungsteil miteinander abschließen und dass der Adapter auf die Stirnseiten der Leitungsenden aufgesetzt ist. In dem anderen Endbereich des Adapters kann ein weiterer Leitungsabschnitt vorgesehen sein.

In weiterer Ausgestaltung der Erfindung ist ein erster und ein zweiter Leitungsteil vorgesehen, wobei der zweite Leitungsteil zweiteilig ausgeführt ist und jeder der Teile stumpf an den Verschlusskörper anschließt, wobei der erste Leitungsteil den zweiten Leitungsteil umgibt und zu beiden Seiten des Verschlusskörpers mit den Teilen des zweiten Leitungsteils fluiddicht in Verbindung steht. Der Bereich zwischen den Teilen des zweiten Leitungsteils und dem Verschlusskörper ist nicht fluiddicht ausgeführt, sondern kann von Medium durchströmt werden. Diese Durchströmung findet auf der Seite des Verschlusskörpers statt, die druckbelastet ist. Das druckführende, in dem zweiten Leitungsteil befindliche Medium durchströmt den Bereich zwischen Stirnseite des zweiten Leitungsteils und der angrenzenden Stirnseite des Verschlusskörpers und gelangt auf diese Weise in den Bereich des ersten Leitungsteils, der den Verschlusskörper umgibt. Dies führt dazu, dass der erste Leitungsteil eine Auswölbung erfährt und sich dadurch in Längsrichtung verkürzt. Dies wiederum führt dazu, dass die Teile des zweiten Leitungsteils mit ihren Stirnseiten auf den Verschlusskörper gepreßt werden, so dass eine zuverlässige Abdichtung entsteht.

Unter dem Begriff des "plastisch verformbaren Verschlusskörpers" bzw. der "plastischen Verformung des Verschlusskörpers" ist nicht nur der Fall zu verstehen, dass der Verschlusskörper plastisch ausgeführt ist, sondern auch der Fall, dass der Verschlusskörper elastisch ausgeführt ist und dass Mittel vorgesehen sind, die den flexiblen bzw. elastischen Verschlusskörper in einer verformten Position fixieren, in der dieser den Leitungsdurchgang zumindest teilweise freigibt. Ist der Verschlusskörper aus flexiblem Material und gelangt der Systemdruck in das Innere des flexiblen Verschlusskörpers, wie das z.B. bei einem einseitig verschlossenen Stück flexibler Leitung der Fall ist, kann sich der Verschlusskörper unter Druck mit der diesen umgebenden flexiblen Leitung dehnen, wodurch der Verschluß dicht bleibt. Damit sich nach dem Verformen des Verschlusskörpers dieser nicht wieder in die ursprüngliche Form begibt, die die Leitung verschließt, muß der flexible Verschlusskörper in der Stellung gehalten werden, in der er den Leitungsdurchgang wenigstens teilweise freigibt. Dazu ist beispielsweise denkbar, dass der flexible bzw. elastische Verschlusskörper mit plastischen Materialien kombiniert ist.

Auch beliebige andere Mittel sind einsetzbar, mit denen das Zurückstellen des verformten elastischen Verschlusskörpers in dessen Schließposition verhindert wird. Denkbar ist z.B., dass zu diesem Zweck Klebstoff vorgesehen ist. Dieser kann z. B. die im verformten Zustand aneinanderliegenden Bereiche des Verschlusskörpers in dieser Position fixieren. Möglich ist z.B., dass der Verschlusskörper hohl ausgeführt ist und auf seiner Innenseite eine Klebeschicht aufweist.

Als Mittel, das den Verschlusskörper in seiner verformten Stellung fixiert, kann ferner ein Klettverschluß vorgesehen sein. Dieser kann beispielsweise auf der Innenseite eines als Hohlkörper ausgeführten Verschlusselementes angeordnet sein und im verformten Zustand sicherstellen, dass die aneinanderliegenden Wandungen des Verschlusskörpers aneinander fixiert werden. Denkbar ist grundsätzlich auch, dass ein Teil des Klettverschlusses an der Leitung und das komplementäre Teil des Klettverschlusses an dem Verschlusskörper angeordnet ist.

Die Erfindung betrifft ferner ein Verfahren zum Öffnen einer Leitung nach einem der Ansprüche 1 bis 15, wobei der Verschlusskörper zum Zwecke der Öffnung der Leitung von dem Zustand, in dem dieser die Leitung verschließt, derart plastisch verformt wird, dass ein Durchlaß durch die Leitung freigegeben wird. Die Verformung findet vorzugsweise von außen, d. h. durch die Leitung hindurch statt.

Die Verformung des Verschlusskörpers zum Zwecke der Freigabe des Durchlasses kann beispielsweise mittels eines Werkzeuges vorgenommen werden. Ein Vorteil der Erfindung besteht darin, dass nicht nur eine manuelle Betätigung sondern auch eine maschinelle Betätigung denkbar ist. Somit kann das Werkzeug Bestandteil einer Maschine oder Apparatur zum Öffnen der Leitung sein. Denkbar ist auch der Einsatz einer handbetätigten Zange.

Bei dem Werkzeug kann es sich um ein Werkzeug mit ebenen Backen handeln. Denkbar ist ebenfalls, dass das Werkzeug eine Backe mit einem Vorsprung und eine Backe mit einer Ausnehmung aufweist, in die der Vorsprung bei geschlossenem Werkzeug wenigstens teilweise eingreift.

Eine Verformung des Verschlusskörpers erfolgt vorzugsweise derart, dass dieser nach seiner Verformung in der Leitung gegen Verschieben fixiert ist. Eine derartige Ausgestaltung der Erfindung erübrigt den Einsatz spezieller Halte- oder Fixierungselemente, die eine Verschiebung des Verschlusskörpers nach dessen Deformation verhindern. Selbstverständlich sind auch Ausgestaltungen der Erfindung denkbar, bei der derartige Arretierungsmittel vorgesehen sind.

Die Erfindung betrifft ferner einen Blutschlauch oder einen Blutbeutel mit einer erfindungsgemäßen Leitung.

Als Materialien für den Verschlusskörper kommen beispielsweise Metalle, wie z.B. Aluminium, oder Kunststoffe, wie z.B. Polyethylen in Betracht. Der Kunststoff kann spröde ausgeführt sein und bei dessen Verformung in einzelne Teile zerkleinert werden.

Soll die Leitung im medizinischen Bereich Verwendung finden, ist vorzugsweise vorgesehen, dass der Verschlusskörper blutkompatibel ist oder eine blutkompatible Beschichtung aufweist.

Das Einbringen des Verschlusskörpers in die Leitung kann beispielsweise dadurch erfolgen, dass der Verschlusskörper mechanisch in die Leitung eingeschoben wird. Um einen guten Sitz und eine gute Dichtheit zu gewährleisten, sollten die Außenabmessungen des Verschlusskörpers geringfügig über den Innenabmessungen der Leitung liegen, so dass die Leitung beim Einschieben geringfügig gedehnt wird und der Verschlusskörper an der gewünschten Position verbleibt und dichtend in der Leitung aufgenommen ist.

Auch ist es denkbar, die Leitung nach dem Einbringen des Verschlusskörpers in die Leitung zumindest in dem Bereich zu schrumpfen, in dem sich der Verschlusskörper befindet. Die Leitung kann somit auch auf den Verschlusskörper aufgeschrumpft werden. Vorteilhaft vollzieht sich der Vorgang der Schrumpfung bei Hitzeeinwirkung. Denkbar ist, dass die Schrumpfung während der Hitzesterilisation erfolgt. Selbstverständlich ist es ebenso möglich, den Vorgang der Schrumpfung unabhängig von der Hitzesterilisation durchzuführen.

Weitere Einzelheiten und Vorteile der Erfindung werden anhand eines in der Zeichnung dargestellten Ausführungsbeispiels näher erläutert. Es zeigen:
- Figuren 1 bis 4:: eine schematische Darstellung der Schritte beim Verformen eines Verschlusskörpers mittels eines Werkzeuges in einer ersten Ausführungsform,
- Figuren 5 bis 8:: eine schematische Darstellung der Schritte beim Verformen eines Verschlusskörpers mittels eines Werkzeuges in einer zweiten Ausführungsform,
- Figur 9:: eine schematische Darstellung einer Leitung mit einem ersten Leitungsteil und einem zweiten Leitungsteil, in dem sich der Verschlusskörper befindet, in einer einseitig druckfesten Ausführung,
- Figur 10:: eine schematische Darstellung der Leitung mit einem ersten Leitungsteil und einem zweiten Leitungsteil, in dem sich der Verschlusskörper befindet, in einer zweiseitig druckfesten Ausführung,
- Figur 11:: eine schematische Darstellung der Leitung mit einem ersten Leitungsteil und einem zweiten Leitungsteil, in dem sich der Verschlusskörper befindet, in einer weiteren einseitig druckfesten Ausführung,
- Figur 12:: eine schematische Darstellung der Leitung mit einem ersten Leitungsteil und einem zweiten Leitungsteil, in dem sich der Verschlusskörper befindet, in einer weiteren einseitig druckdichten Ausführung mit Adapter,
- Figuren 13, 14:: schematische Darstellungen der Leitung mit einem ersten Leitungsteil und einem zweiten Leitungsteil, der zweiteilig ausgeführt ist, in einer zweiseitig druckdichten Ausführung vor Druckbeaufschlagung (Figur 13) und bei Druckbeaufschlagung (Figur 14) und
- Figur 15:: eine Querschnittsansicht der Leitung mit verformtem Verschlusskörper mit Detaildarstellungen.

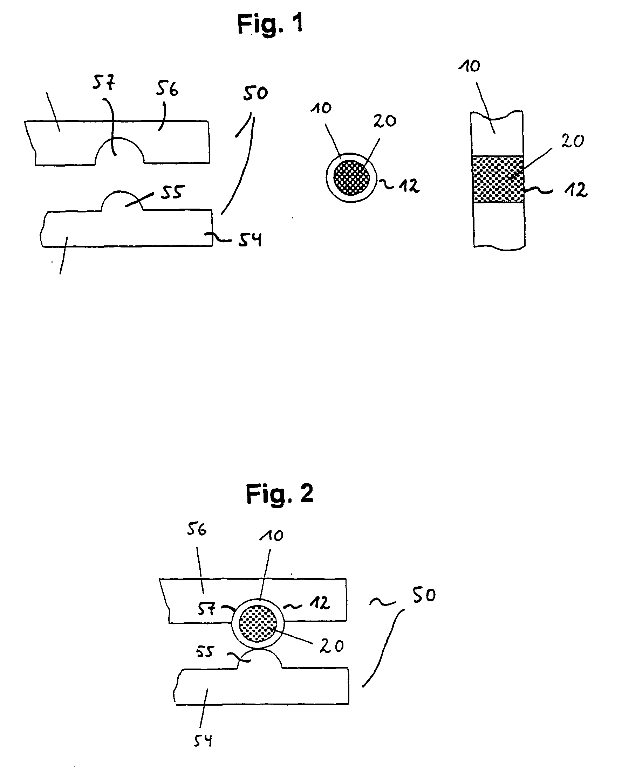

Figur 1, linke Darstellung zeigt ein manuell oder maschinell zu betätigendes Werkzeug 50, das eine Backe 54 mit einem halbkreisförmigen Vorsprung 55 und eine Backe 56 aufweist, in der eine ebenfalls halbkreisförmige Ausnehmung 57 angeordnet ist, wobei der Durchmesser der Ausnehmung 57 über dem des Vorsprungs 55 liegt.

Figur 1, mittlere Darstellung zeigt in einer schematischen Querschnittsansicht die erfindungsgemäße Leitung 10, die durch den Verschlusskörper 20 verschlossen ist. Der Verschlusskörper 20 befindet sich in dem Abschnitt 12 der Leitung 10, der flexibel ausgeführt ist. Denkbar sind Ausführungsformen, bei denen nur der Abschnitt 12 flexibel ausgeführt ist, in dem sich der Verschlusskörper 20 befindet. Denkbar ist ebenfalls, dass die gesamte Leitung 10 flexibel ausgeführt ist.

Der Verschlusskörper 20 kann massiv, hohl, nach einer Seite der Leitung offen oder vollumfänglich geschlossen ausgeführt sein. Er kann aus porösem und undurchlässigem Material bestehen oder auch hohl ausgeführt sein und eine Füllung aufweisen.

Um die Haftung zwischen dem Verschlusskörper 20 und der Innenseite der Wandung der Leitung im Abschnitt 12 zu verbessern, kann vorgesehen sein, dass zwischen beiden Elementen Klebstoff angeordnet ist, der so dosiert sein muß, dass die gewünschte Dichtwirkung erzielt wird, die Verbindung jedoch für den Fall, dass der Durchlaß durch die Leitung 10 geöffnet werden soll, dennoch lösbar bleibt.

Figur 1, rechte Darstellung zeigt in einer schematischen Längsschnittansicht die erfindungsgemäße Leitung 10, in deren Abschnitt 12 der Verschlusskörper 20 derart aufgenommen ist, dass er die Leitung 10 verschließt.

Figur 2 zeigt die Aufnahme der Leitung 10 in dem Werkzeug 50. Wie dies aus Figur 2 ersichtlich ist, entspricht der Radius der Ausnehmung 57 der Backe 56 des Werkzeuges 50 in etwa dem Außendurchmesser des Abschnitts 12 der Leitung 10. Die Leitung 10 wird in der Ausnehmung 57 mittels des Vorsprungs 55 fixiert, wie dies aus Figur 2 hervorgeht.

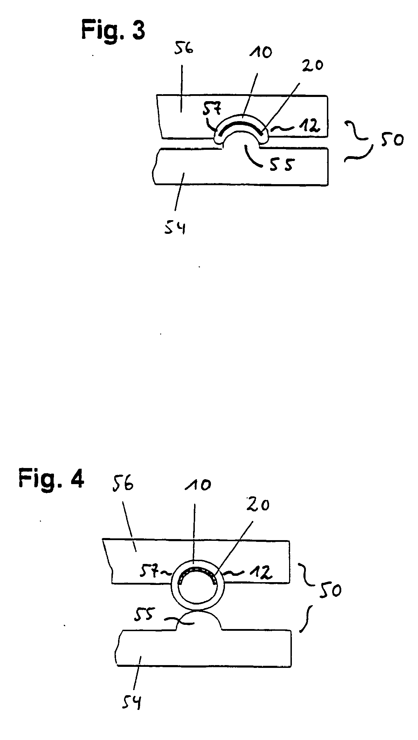

Wird das Werkzeug 50 maschinell oder manuell betätigt, das heißt werden die Bakken 54, 56 zusammengeführt, wird der zwischen den Backen 54, 56 befindliche Leitungsabschnitt gequetscht, wie dies in Figur 3 angedeutet ist. Dabei wird der Verschlusskörper 20 plastisch verformt. Aufgrund der Ausführung der Ausnehmung 57 und des Vorsprungs 55 ergibt sich beim Verformen eine teilkreisförmige Gestalt des Verschlusskörpers 20, wie dies aus Figur 3 hervorgeht. Die Kraft zur Verformung des Verschlusskörpers 20 wird durch die Wandung der Leitung 10 übertragen.

Figur 4 zeigt die Anordnung nach dem Öffnen des Werkzeuges 50. Wie dies aus Figur 4 ersichtlich ist, verbleibt der Verschlusskörper 20 in seiner verformten Position und gibt dadurch dauerhaft einen Durchlaß durch die Leitung 10 frei. Die Leitung 10 bzw. deren Abschnitt 12 nimmt aufgrund der flexiblen Ausführung wieder die ursprüngliche Gestalt vor dem Verformungsvorgang ein.

Die Leitung 10 bzw. deren Abschnitt weist vor und nach der Verformung dieselbe Form auf.

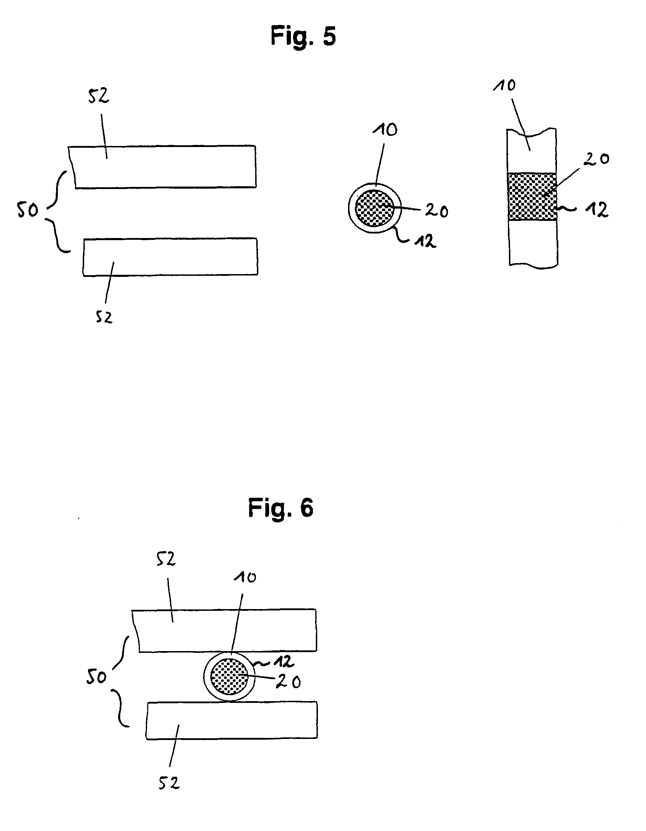

Figur 5 zeigt ein Werkzeug 50 mit eben ausgeführten Backen 52. Die Ausführung der Leitung 10 mit Verschlusskörper 20 entspricht dem Ausführungsbeispiel zu den Figuren 1 bis 4.

Figur 6 zeigt die Positionierung der Leitung 10 mit Verschlusskörper 20 zwischen den Backen 52 des Werkzeuges 50.

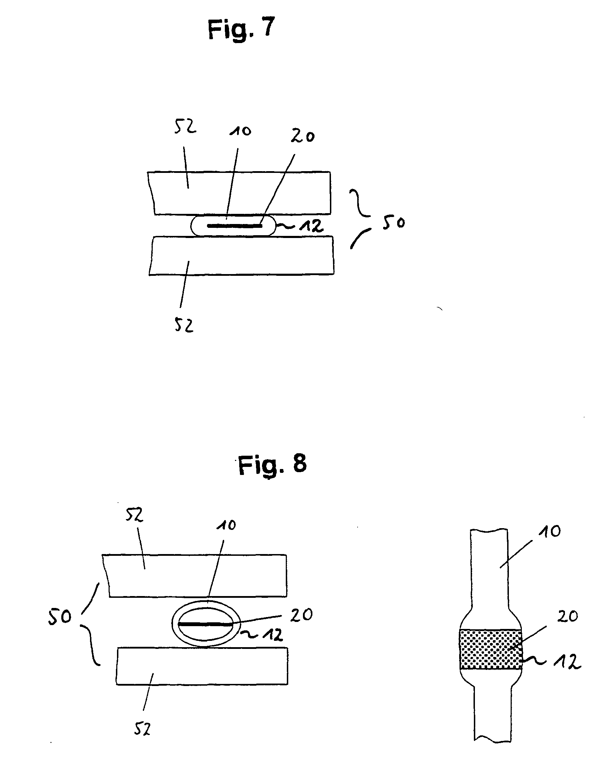

In Figur 7 ist der Zustand dargestellt, der sich während des Zusammendrückens der Backen 52 des Werkzeuges 50 ergibt. Beim Zusammendrücken wird der Verschlusskörper 20 in eine Form gebracht, die diesen nach Öffnen des Werkzeuges 50 an Ort und Stelle in dem Abschnitt 12 der Leitung 10 hält und trotzdem einen großen Durchlaß in der Leitung 10 freigibt. Die Kraft zur Verformung des Verschlusskörpers 20 wird durch die Wandung der zumindest in dem Abschnitt 12 flexiblen Leitung 10 übertragen.

Figur 8 zeigt die Anordnung nach dem Öffnen des Werkzeuges. Wie dies aus Figur 8, linke Darstellung ersichtlich ist, ist der Verschlusskörper 20 dauerhaft verformt und nimmt die Form eines Streifens an. Der Verschlusskörper 20 teilt den Abschnitt 12 der Leitung 10 in zwei Durchlassbereiche oberhalb und unterhalb des verformten Verschlusskörpers 20. Der Verschlusskörper 20 befindet sich nach der Verformung dauerhaft in einer Form, die diesen an Ort und Stelle fixiert. Dazu wird, wie dies auch aus dem schematischen Längsschnitt gemäß Figur 8, rechte Darstellung ersichtlich ist, der flexible Teil 12 der Leitung 10 durch die Form des Verschlusskörpers 20 gedehnt, was eine Rückstellkraft des Abschnitts 12 der Leitung 10 zur Folge hat. Diese Kraft hält den verformten Verschlusskörper in seiner neuen Form an Ort und Stelle.

Werden hohe Anforderungen an die Druckfestigkeit des Verschlusses der flexiblen Leitung gestellt, sind Ausführungen möglich, die konstruktiv eine beliebige Druckfestigkeit ermöglichen. Dazu ist vorgesehen, dass die erste flexible Leitung durch eine zweite flexible Leitung ergänzt wird, die so angeordnet ist, dass die zweite flexible Leitung zumindest abschnittsweise innerhalb der ersten flexiblen Leitung verläuft.

Dabei ist vorzugsweise vorgesehen, dass die erste und die zweite Leitung so miteinander verbunden sind, dass zwischen beiden Leitungen ein ringförmiger, umlaufender Verschluß entsteht. Im Inneren der zweiten Leitung befindet sich der Verschlusskörper. Beide Leitungen sind vorzugsweise flexibel ausgeführt.

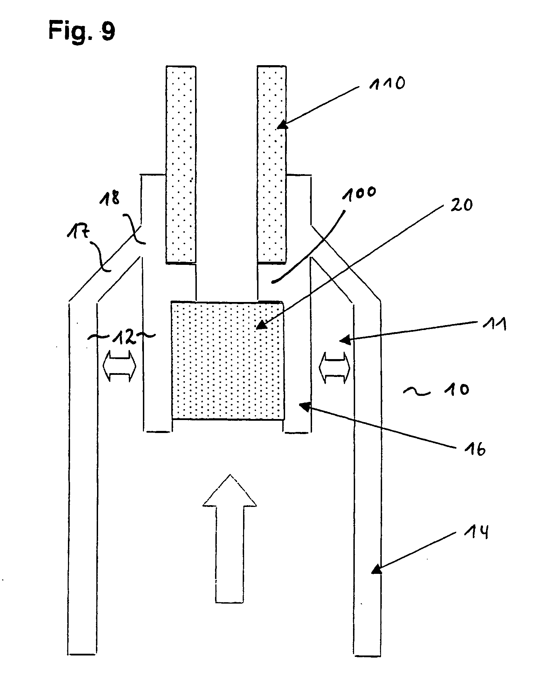

Figur 9 zeigt ein Ausführungsbeispiel der Erfindung in dieser Ausführungsform. Die Leitung 10 umfaßt einen ersten Leitungsteil 14 und einen zweiten Leitungsteil 16, wobei sich der Verschlusskörper 20 in dem zweiten Leitungsteil 16 derart befindet, dass er diesen verschließt. Der zweite Leitungsteil 16 weist einen nach innen ragenden Steg 100 auf, an dem einerseits der Verschlusskörper 20 und andererseits ein weiterer Leitungsteil 110 anliegt, der die Verbindung zu weiteren Gefäßen oder dem Auslauf darstellt.

Wie dies aus Figur 9 ersichtlich ist, sind der erste 14 und der zweite Leitungsteil 16 in dem Bereich des zweiten Leitungsteils 16, in dem sich der Verschlusskörper 20 befindet, derart zueinander angeordnet, dass der erste Leitungsteil 14 den zweiten Leitungsteil 16 konzentrisch umgibt. Zwischen beiden Leitungsteilen 14, 16 ergibt sich in diesem Bereich ein ringförmiger Raum 11, d.h. beide Leitungsteile 14, 16 sind in diesem Bereich radial voneinander beabstandet. Wirkt in dem ersten Leitungsteil 14 ein Druck, wie dies durch den vertikal angeordneten Pfeil angedeutet ist, führt dieser dazu, dass entsprechend der Doppelpfeile in dem Raum 11 auf den Abschnitt des zweiten Leitungsteils 16 eine zum Verschlusskörper 20 gerichtete Kraft ausgeübt wird, wodurch ein Öffnen des Verschlusses unabhängig vom Innendruck vollständig unterbunden wird.

An dem zylindrisch verlaufenden Abschnitt des ersten Leitungsteils 14 schließt sich der konisch zulaufende Bereich 17 an, der den zylindrischen Abschnitt des ersten Leitungsteils 14 mit dem zweiten Leitungsteil 16 verbindet. Im Endbereich des konischen Abschnittes 17 sind die beiden Leitungsteile 14, 16 unter Bildung der Verbindungsstelle 18 fluiddicht miteinander verbunden.

Denkbar ist, dass im nicht druckbeaufschlagten Zustand der erste Leitungsteil 14 an dem zweiten Leitungsteil 16 anliegt und sich erst im druckbeaufschlagten Zustand die in Figur 9 dargestellte Position ergibt. Denkbar ist ferner eine Ausführungsform, bei der sich auch im nicht druckbeaufschlagten Zustand unter Bildung des Ringraumes 11 ein Abstand zwischen beiden Leitungsteilen 14, 16 bildet.

Durch die Positionierung des Verschlusskörpers im Bezug auf die umlaufende Verbindung des ersten und zweiten Leitungsteils 14, 16 kann festgelegt werden, ob die Druckfestigkeit von einer oder von beiden Seiten des Verschlusskörpers 20 gegeben ist.

Wie oben ausgeführt, sind die Leitungsteile 14, 16 zumindest im Bereich des Verschlusskörpers flexibel ausgeführt.

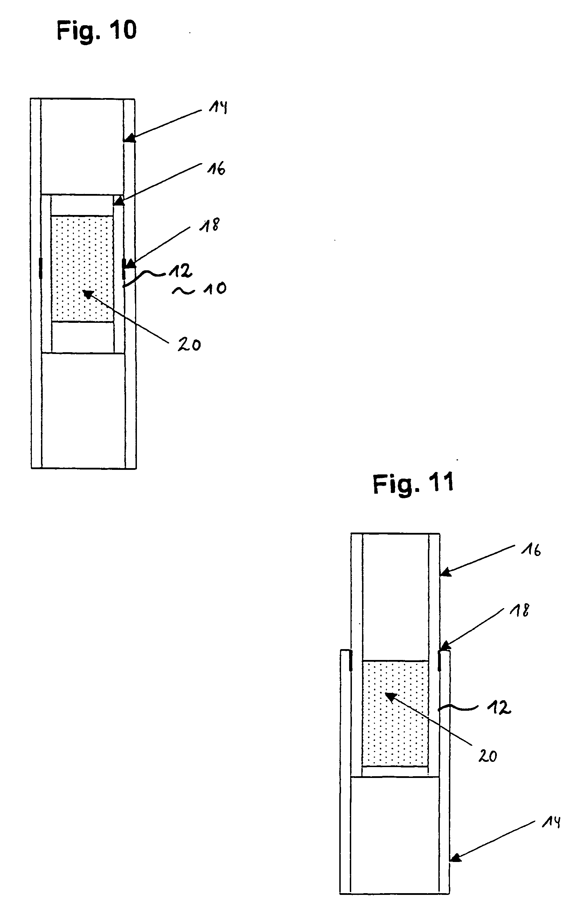

Figur 10 zeigt eine Ausführung der erfindungsgemäßen Leitung 10, bei der der zweite Leitungsteil 16 vollständig von dem ersten Leitungsteil 14 umgeben ist, wobei im drucklosen Zustand beide Leitungsteile 14, 16 aneinander anliegen, wie dies in Figur 10 verdeutlicht ist. In dem zweiten Leitungsteil 16 ist diesen verschließend der Verschlusskörper 20 aufgenommen. An der umlaufenden Verbindungsstelle 18 sind die beiden Leitungsteile 14, 16 miteinander fest und fluiddicht verbunden. Wird der erste Leitungsteil 14 aufgrund des darin herrschenden Innendruckes gedehnt, so entsteht zwischen den Leitungsteilen 14, 16 ein Spalt, in dem sich der Druck so ausbreiten kann, dass er den zweiten Leitungsteil 16 auf den Verschlusskörper 20 preßt, wodurch eine zuverlässige Abdichtung erzielt wird.

Die Verbindungsstelle 18 zwischen den Leitungsteilen 14, 16 befindet sich in etwa mittig an dem zweiten Leitungsteil 16. Ebenfalls in etwa zentrisch im zweiten Leitungsteil 16 ist der Verschlusskörper 20 aufgenommen, so dass sich insgesamt eine zweiseitig druckdichte Ausführung ergibt.

Figur 11 zeigt eine einseitig druckdichte Ausführung, bei der der zweite Leitungsteil 16 teilweise innerhalb des ersten Leitungsteils 14 aufgenommen ist und teilweise über diesen übersteht. Im Endbereich des ersten Leitungsteils 14 befindet sich die Verbindungsstelle 18, die die Leitungsteile 14, 16 fest und fluiddicht miteinander verbindet. In dem Abschnitt, in dem der zweite Leitungsteil 16 in dem ersten Leitungsteil 14 aufgenommen ist, befindet sich in dem zweiten Leitungsteil 16 der Verschlusskörper 20. Wird der erste Leitungsteil 14 mit Druck beaufschlagt, führt dies dazu, dass sich zwischen beiden Leitungsteilen 14, 16 ein Spalt ergibt, wobei der in dem Spalt herrschende Druck den den Spalt begrenzenden Bereich des zweiten Leitungsteils 16 auf den Verschlusskörper 20 preßt.

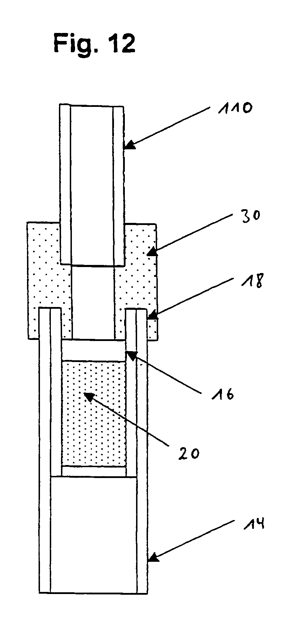

Figur 12 zeigt eine weitere Ausführungsform einer einseitig druckdichten Ausführung, bei der die Leitungsteile 14, 16 bündig miteinander abschließen. In diesem Endbereich der Leitungsteile 14, 16 ist ein Adapter 30 vorgesehen, der auf die Endbereiche der Leitungsteile 14, 16 aufgesteckt ist und diese fluiddicht unter Bildung der Verbindungsstelle 18 miteinander verpresst. Denkbar ist auch, dass der Adapter 30 mit den Leitungsteilen 14, 16 mittels Klebung verbunden ist. Auch aus diesem Ausführungsbeispiel ergibt sich, dass sich bei Druckbeaufschlagung des ersten Leitungsteils 14 zwischen den beiden Leitungsteilen 14, 16 ein Spalt bzw. ein Ringraum ergibt, wobei der in diesem herrschende Druck den zweiten Leitungsteil 16 auf den Verschlusskörper 20 preßt.

Mit dem Bezugszeichen 110 ist eine Leitung dargestellt, die zu weiteren Gefäßen oder einem Auslauf verläuft und fluiddicht mit dem Adapter 30 in Verbindung steht.

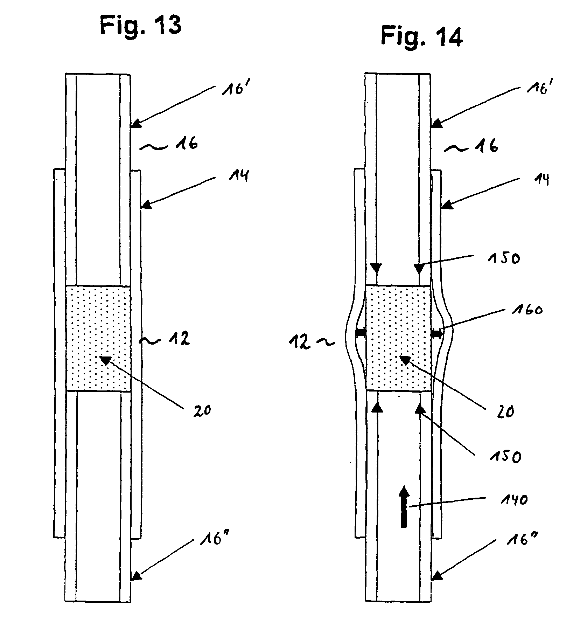

Die Figuren 13 und 14 zeigen ein weiteres Ausführungsbeispiel der vorliegenden Erfindung in einer zweiseitig druckdichten Ausführung. In diesem Ausführungsbeispiel ist der zweite Leitungsteil 16 zweiteilig ausgeführt, wobei beide Teile 16', 16" des zweiten Leitungsteils 16 stumpf an den zwischen diesen angeordneten Verschlusskörper 20 angrenzen. Dabei fluchten die Außenseiten der beiden Teile des zweiten Leitungsteils 16 mit der Außenseite des Verschlusskörpers 20. Des weiteren ist ein erster Leitungsteil 14 vorgesehen, der konzentrisch den Verschlusskörper 20 sowie die Bereiche der Teile des zweiten Leitungsteils 16 umgibt, die an den Verschlusskörper 20 angrenzen. Zu beiden Seiten des Verschlusskörpers 20 sind die Teile des zweiten Leitungsteils 16 fluiddicht mit dem ersten Leitungsteil 14 verbunden.

Wird die Anordnung gemäß Figur 13 mit Druck beaufschlagt, wie dies in Figur 14 mit dem vertikal angeordneten Pfeil 140 angedeutet ist, führt dies dazu, dass das druckführende Medium zwischen der Stirnseite eines Teils 16" des zweiten Leitungsteils 16 und der angrenzenden Stirnseite des Verschlusskörpers 20 hindurchströmt und zu einer Auswölbung des ersten Leitungsteils 14 führt, wie dies aus Figur 14 ersichtlich ist. Dadurch ergibt sich ein gewölbter Ringraum zwischen der Außenseite des Verschlusskörpers 20 und der Innenseite des benachbarten Leitungsteils 14. Die durch die Druckbeaufschlagung bedingte Auswölbung (Pfeile 160) des ersten Leitungsteils 14 in dem Bereich, in dem sich der Verschlusskörper 20 befindet führt dazu, dass beide Teile 16', 16" des zweiten Leitungsteils 16 die durch Pfeile 150 angedeutete Kraft in Richtung auf den Verschlusskörper 20 erfahren, was dazu führt, dass die Teile des zweiten Leitungsteils 16 mit ihren Stirnseiten auf die angrenzenden Stirnseiten des Verschlusskörpers 20 gepreßt werden und die Abdichtung sicherstellen.

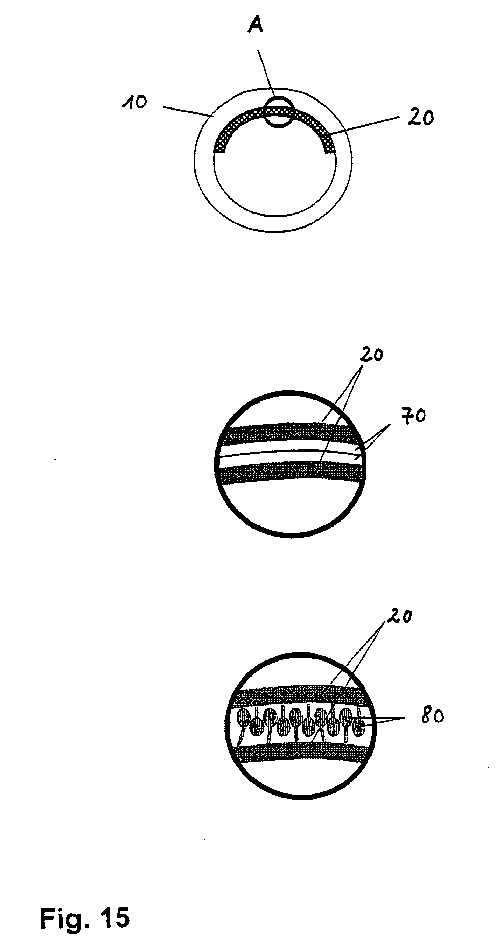

Fig. 15, oben zeigt eine Querschnittsansicht der erfindungsgemäßen Leitung 10 mit dem darin aufgenommenen, verformten Verschlusskörper 20. Wie dies aus Fig. 15, oben ersichtlich ist, gibt der verformte Verschlusskörper 20 einen wesentlichen Teil des Leitungsquerschnittes frei.

Wie dies oben ausgeführt ist, kann der Verschlusskörper 20 aus einem plastischen oder auch aus einem elastischen Material bestehen. Auch Kombinationen elastischer und plastischer Materialien sind einsetzbar.

Ist der Verschlusskörper 20 elastisch bzw. flexibel, ist sicherzustellen, dass dieser nach seiner Verformung nicht wieder die ursprüngliche, die Leitung 10 verschließende Form einnimmt. Hierzu sind beliebige Mittel denkbar, die den flexiblen Verschlusskörper 20 in seiner verformten Position halten.

Die Abbildungen gemäß Fig. 15, mittlere und untere Darstellung, sind Detaildarstellungen des Details A in Fig. 15, oben, die zwei unterschiedliche Alternativen offenbaren, wie der flexible Verschlusskörper 20 in der verformten Position gehalten werden kann.

Fig. 15, mittlere Darstellung zeigt die zwei Wandungen des Verschlusskörpers 20, die jeweils mit einer Klebeschicht 70 versehen sind. Werden diese Klebeschichten 70 aneinander geführt, wie dies beim Verformen des Verschlusskörpers 20 der Fall ist und wie dies in Fig. 15, mittlere Darstellung dargestellt ist, führt dies dazu, dass der Verschlusskörper 20 in seiner in Fig. 15 dargestellten verformten Position fixiert wird. Fig. 15, untere Darstellung zeigt eine alternative Ausführungsform, bei der die Innenseiten der Wandungen des Verschlusskörpers 20 mit einem Klettverschluß 80 versehen sind, der ebenfalls bewirkt, dass der verformte Verschlusskörper 20 in seiner verformten Position verbleibt.

In dem in Fig. 15 dargestellten Ausführungsbeispiel ist der Verschlusskörper 20 beispielsweise als einseitig offener Hohlkörper ausgeführt, dessen innere Wandungen mit der Klebeschicht 70 bzw. mit dem Klettverschluß 80 versehen sind.

Selbstverständlich sind auch andere Mittel als die dargestellten einsetzbar, um sicherzustellen, dass der Verschlusskörper 20 in seiner verformten Position verbleibt.

## Patentansprüche

1. Leitung (10), insbesondere Leitung eines Blutschlauch- oder Blutbeutelsystems, mit einem den Leitungsdurchlaß absperrenden Verschlusselement, **dadurch gekennzeichnet, dass** das Verschlusselement durch einen in der Leitung (10) angeordneten, plastisch verformbaren Verschlusskörper (20) gebildet wird, der in wenigstens einem Zustand die Leitung (10) verschließt, und dass wenigstens der Abschnitt (12) der Leitung (10), in dem sich der Verschlusskörper (20) befindet, flexibel ausgeführt ist.

2. Leitung (10) nach Anspruch 1, **dadurch gekennzeichnet, dass** der Verschlusskörper (20) massiv, porös oder als Hohlkörper ausgeführt ist, der ein- oder mehrseitig offen oder der geschlossen ausgeführt ist.

3. Leitung (10) nach Anspruch 1 oder 2, **dadurch gekennzeichnet, dass** der Verschlusskörper (20) als Hohlkörper ausgeführt ist, in dem sich eine Füllung befindet.

4. Leitung (10) nach einem der vorhergehenden Ansprüche, **dadurch gekennzeichnet, dass** der Verschlusskörper (20) sowie der Abschnitt (12) der Leitung (10), in dem sich der Verschlusskörper (20) befindet, aus Materialien bestehen oder Materialien aufweisen, die aneinander haften.

5. Leitung (10) nach einem der vorhergehenden Ansprüche, **dadurch gekennzeichnet, dass** sich zwischen dem Verschlusskörper (20) sowie dem Abschnitt (12) der Leitung (10), in dem sich der Verschlusskörper (20) befindet, Klebstoff zur Verbesserung der Haftung zwischen beiden Bauteilen (12, 20) befindet.

6. Leitung (10) nach einem der vorhergehenden Ansprüche, **dadurch gekennzeichnet, dass** die Oberfläche des Verschlusskörpers (20) und/oder die innen befindliche Oberfläche des Abschnittes (12) der Leitung (10), in dem sich der Verschlusskörper (20) befindet, porös bzw. rauh ausgeführt sind.

7. Leitung (10) nach einem der vorhergehenden Ansprüche, **dadurch gekennzeichnet, dass** die Leitung (10) einen ersten (14) und einen zweiten Leitungsteil (16) aufweist, die an wenigstens einer Verbindungsstelle (18) fluiddicht miteinander in Verbindung stehen, wobei der Verschlusskörper (20) in wenigstens einem Zustand den zweiten Leitungsteil (16) verschließt und wobei der erste (14) und der zweite Leitungsteil (16) wenigstens abschnittsweise in dem Bereich des zweiten Leitungsteils (16), in dem sich der Verschlusskörper (20) befindet, derart angeordnet sind, dass der erste Leitungsteil (14) den zweiten Leitungsteil (16) umgibt und beide Leitungsteile (14, 16) in diesem Bereich wenigstens abschnittsweise voneinander beabstandet oder voneinander lösbar sind.

8. Leitung (10) nach Anspruch 7, **dadurch gekennzeichnet, dass** der erste (14) und zweite Leitungsteil (16) zueinander konzentrisch angeordnet sind.

9. Leitung (10) nach Anspruch 7 oder 8, **dadurch gekennzeichnet, dass** in dem Abschnitt (12) des zweiten Leitungsteils (16), in dem der Verschlusskörper (20) angeordnet ist, beide Leitungsteile (14, 16) voneinander beabstandet sind, und dass in einem hierzu benachbarten Bereich beide Leitungsteile (14, 16) mittels eines vorzugsweise konisch verlaufenden Verbindungsabschnittes (17) miteinander verbunden sind.

10. Leitung (10) nach einem der Ansprüche 7 bis 9, **dadurch gekennzeichnet, dass** der zweite Leitungsteil (16) in dem ersten Leitungsteil (14) aufgenommen ist und dass die Verbindungsstelle (18) in dem Randbereich des zweiten Leitungsteils (16) oder davon beabstandet, vorzugsweise mittig angeordnet ist.

11. Leitung (10) nach einem der Ansprüche 7 bis 10, **dadurch gekennzeichnet, dass** der zweite Leitungsteil (16) teilweise in dem ersten Leitungsteil (14) angeordnet ist und teilweise über diesen vorsteht und dass die Verbindungsstelle (18) im Endbereich des ersten Leitungsteils (14) angeordnet ist.

12. Leitung (10) nach einem der Ansprüche 7 bis 11, **dadurch gekennzeichnet, dass** ein Adapter (30) vorgesehen ist, der auf den ersten (14) und zweiten Leitungsteil (16) aufgesteckt ist.

13. Leitung (10) nach einem der vorhergehenden Ansprüche, **dadurch gekennzeichnet, dass** ein erster (14) und ein zweiter Leitungsteil (16) vorgesehen sind, wobei der zweite Leitungsteil (16) zweiteilig ausgeführt ist und jeder der Teile (16', 16") stumpf an den Verschlusskörper (20) anschließt, wobei der erste Leitungsteil (14) den zweiten Leitungsteil (16) umgibt und zu beiden Seiten des Verschlusskörpers (20) mit den Teilen (16', 16") des zweiten Leitungsteils (16) fluiddicht in Verbindung steht.

14. Leitung (10) nach einem der vorhergehenden Ansprüche, **dadurch gekennzeichnet, dass** der Verschlusskörper (20) aus einem oder mehreren elastischen und/oder plastischen Materialien besteht.

15. Leitung (10) nach einem der vorhergehenden Ansprüche, **dadurch gekennzeichnet, dass** der Verschlusskörper (20) elastisch ist und dass Mittel vorgesehen sind, die den Verschlusskörper (20) in einer verformten Position fixieren.

16. Verfahren zum Öffnen einer Leitung nach einem der Ansprüche 1 bis 15, **dadurch gekennzeichnet, dass** der Verschlusskörper (20) zum Zwecke der Öffnung der Leitung (10) von dem Zustand, in dem dieser die Leitung (10) verschließt, derart plastisch verformt wird, dass ein Durchlaß durch die Leitung (10) freigegeben wird.

17. Verfahren nach Anspruch 16, **dadurch gekennzeichnet, dass** die Verformung des Verschlusskörpers (20) mittels eines Werkzeuges (50) vorgenommen wird.

18. Verfahren nach Anspruch 17, **dadurch gekennzeichnet, dass** es sich bei dem Werkzeug (50) um eine handbetätigte Zange oder um den Bestandteil einer Maschine handelt.

19. Verfahren nach Anspruch 17 oder 18, **dadurch gekennzeichnet, dass** das Werkzeug (50) ebene Backen (52) aufweist.

20. Verfahren nach Anspruch 17 oder 18, **dadurch gekennzeichnet, dass** das Werkzeug (50) eine Backe (54) mit einem Vorsprung (55) und eine Backe (56) mit einer Ausnehmung (57) aufweist, in die der Vorsprung (55) bei geschlossenem Werkzeug (50) wenigstens teilweise eingreift.

21. Verfahren nach einem der Ansprüche 16 bis 20, **dadurch gekennzeichnet, dass** die Verformung des Verschlusskörpers (20) derart erfolgt, dass dieser nach seiner Verformung in der Leitung (10) gegen Verschieben fixiert ist.

22. Blutschlauch oder Blutbeutel mit einer Leitung nach einem der Ansprüche 1 bis 15.

## Claims

1. A line (10), in particular a line of a blood hose or blood bag system, comprising a closure element for closing off the passage through the line, **characterised in that** the closure element is formed by a plastically deformable closure body (20) which is arranged in the line (10) and which closes the line (10) in at least one condition, and that at least the portion (12) of the line (10), in which the closure body (20) is disposed, is flexible.

2. A line (10) according to claim 1 **characterised in that** the closure body (20) is solid, porous or in the form of a hollow body which at one or more sides is open or is closed.

3. A line (10) according to claim 1 or claim 2 **characterised in that** the closure body (20) is in the form of a hollow body in which there is a filling.

4. A line (10) according to one of the preceding claims **characterised in that** the closure body (20) and the portion (12) of the line (10), in which the closure body (20) is disposed, comprise or have materials which adhere to each other.

5. A line (10) according to one of the preceding claims **characterised in that** between the closure body (20) and the portion (12) of the line (10) in which the closure body (20) is disposed is adhesive for improving the adhesion between the two components (12, 20).

6. A line (10) according to one of the preceding claims **characterised in that** the surface of the closure body (20) and/or the inwardly disposed surface of the portion (12) of the line (10), in which the closure body (20) is disposed, is porous or rough.

7. A line (10) according to one of the preceding claims **characterised in that** the line (10) has a first line part (14) and a second line part (16) which are fluid-tightly connected together at at least one connecting location (18), wherein the closure body (20) closes the second line part (16) in at least one condition and wherein the first line part (14) and the second line part (16) are arranged at least portion-wise in the region of the second line part (16) in which the closure body (20) is disposed, in such a way that the first line part (14) surrounds the second line part (16) and both line parts (14, 16) in said region are at least portion-wise spaced from each other or releasable from each other.

8. A line (10) according to claim 7 **characterised in that** the first line part (14) and the second line part (16) are arranged concentrically relative to each other.

9. A line (10) according to claim 7 or claim 8 **characterised in that** both line parts (14, 16) are spaced from each other in the portion (12) of the second line part (16) in which the closure body (20) is arranged and that in a region adjacent thereto both line parts (14, 16) are connected together by means of a preferably conically extending connecting portion (17).

10. A line (10) according to one of claims 7 to 9 **characterised in that** the second line part (16) is received in the first line part (14) and that the connecting location (18) is arranged in the edge region of the second line part (16) or spaced therefrom, preferably centrally.

11. A line (10) according to one of claims 7 to 10 **characterised in that** the second line part (16) is partially arranged in the first line part (14) and partially projects beyond same and that the connecting location (18) is arranged in the end region of the first line part (14).

12. A line (10) according to one of claims 7 to 11 **characterised in that** there is provided an adaptor (30) which is fitted on to the first line part (14) and the second line part (16).

13. A line (10) according to one of the preceding claims **characterised in that** there are provided a first line part (14) and a second line part (16), wherein the second line part (16) is of a two-part configuration and each of the parts (16', 16") adjoins the closure body (20) in butting relationship, wherein the first line part (14) surrounds the second line part (16) and at both sides of the closure body (20) is fluid-tightly connected to the parts (16', 16") of the second line part (16).

14. A line (10) according to one of the preceding claims **characterised in that** the closure body (20) comprises one or more elastic and/or plastic materials.

15. A line (10) according to one of the preceding claims **characterised in that** the closure body (20) is elastic and that there are provided means which fix the closure body (20) in a deformed position.

16. A method of opening a line according to one of claims 1 to 15 **characterised in that** for the purposes of opening the line (10) the closure body (20) is plastically deformed from the condition in which it closes the line (10) in such a way that a passage through the line (10) is opened.

17. A method according to claim 16 **characterised in that** the deformation of the closure body (20) is effected by means of a tool (50).

18. A method according to claim 17 **characterised in that** the tool (50) is a hand-actuated pincer means or the component of a machine.

19. A method according to claim 17 or claim 18 **characterised in that** the tool has flat jaws (52).

20. A method according to claim 17 or claim 18 **characterised in that** the tool (50) has a jaw (54) having a projection (55) and a jaw (56) having a recess (57) into which the projection (55) at least partially engages when the tool (50) is closed.

21. A method according to one of claims 16 to 20 **characterised in that** deformation of the closure body (20) is effected in such a way that after its deformation it is fixed against displacement in the line (10).

22. A blood hose or blood bag having a line according to one of claims 1 to 15.

## Revendications

1. Conduite (10), en particulier conduite pour un système de tube de prélèvement sanguin ou de poche de sang, avec un élément de fermeture fermant le passage de conduite, **caractérisée en ce que** l'élément de fermeture est formé par un corps de fermeture (20) plastiquement déformable, disposé dans la conduite (10), qui ferme la conduite (10) dans au moins un état, et **en ce qu'**au moins la section (12) de la conduite (10), dans laquelle se trouve le corps de fermeture (20), est réalisée d'une manière flexible.

2. Conduite (10) selon la revendication 1, **caractérisée en ce que** le corps de fermeture (20) est réalisé d'une manière massive, poreuse ou comme corps creux, qui est réalisé en étant ouvert sur un ou plusieurs côtés ou en étant fermé.

3. Conduite (10) selon la revendication 1 ou 2, **caractérisée en ce que** le corps de fermeture (20) est réalisé comme corps creux, dans lequel se trouve une matière de remplissage.

4. Conduite (10) selon l'une des revendications précédentes, **caractérisée en ce que** le corps de fermeture (20) ainsi que la section (12) de la conduite (10), dans laquelle se trouve le corps de fermeture (20), sont constitués de matériaux ou présentent des matériaux qui adhèrent les uns aux autres.

5. Conduite (10) selon l'une des revendications précédentes, **caractérisée en ce qu'**il se trouve entre le corps de fermeture (20) et la section (12) de la conduite (10), dans laquelle se trouve le corps de fermeture (20), une colle pour améliorer l'adhérence entre les deux composants (12, 20).

6. Conduite (10) selon l'une des revendications précédentes, **caractérisée en ce que** la surface du corps de fermeture (20) et/ou la surface se trouvant à l'intérieur de la section (12) de la conduite (10), dans laquelle se trouve le corps de fermeture (20), sont réalisées d'une manière poreuse respectivement rugueuse.

7. Conduite (10) selon l'une des revendications précédentes, **caractérisée en ce que** la conduite (10) présente une première (14) et une deuxième partie de conduite (16), qui sont reliées d'une manière étanche au fluide à au moins un emplacement de liaison (18), où le corps de fermeture (20) ferme dans au moins un état la deuxième partie de conduite (16), et où la première (14) et la deuxième partie de conduite (16) sont disposées au moins par section dans la zone de la deuxième partie de conduite (16), dans laquelle se trouve le corps de fermeture (20), de façon que la première partie de conduite (14) entoure la deuxième partie de conduite (16) et que les deux parties de conduite (14, 16), dans cette zone, sont espacées au moins par section l'une de l'autre ou peuvent être détachées l'une de l'autre.

8. Conduite (10) selon la revendication 7, **caractérisée en ce que** la première (14) et deuxième partie de conduite (16) sont disposées d'une manière concentrique l'une par rapport à l'autre.

9. Conduite (10) selon la revendication 7 ou 8, **caractérisée en ce que** dans la section (12) de la deuxième partie de conduite (16), dans laquelle est disposé le corps de fermeture (20), les deux parties de conduite (14, 16) sont espacées l'une de l'autre, et **en ce que** dans une zone avoisinant celle-ci, les deux parties de conduite (14, 16) sont reliées l'une à l'autre au moyen d'une section de liaison s'étendant de préférence d'une manière conique.

10. Conduite (10) selon l'une des revendications 7 à 9, **caractérisée en ce que** la deuxième partie de conduite (16) est reçue dans la première partie de conduite (14), et **en ce que** l'emplacement de liaison (18) est disposé dans la zone de bord de la deuxième partie de conduite (16) ou à distance de celle-ci, de préférence au milieu.

11. Conduite (10) selon l'une des revendications 7 à 10, **caractérisée en ce que** la deuxième partie de conduite (16) est disposée partiellement dans la première partie de conduite (14) et fait saillie en partie sur celle-ci, et **en ce que** l'emplacement de liaison (18) est disposé dans la zone d'extrémité de la première partie de conduite (14).

12. Conduite (10) selon l'une des revendications 7 à 11, **caractérisée en ce qu'**un adaptateur (30) est prévu qui est emboîté sur la première (14) et deuxième partie de conduite (16).

13. Conduite (10) selon l'une des revendications précédentes, **caractérisée en ce qu'**une première (14) et une deuxième partie de conduite (16) sont prévues, où la deuxième partie de conduite (16) est réalisée en deux parties et chacune des parties (16', 16") étant bout à bout avec le corps de fermeture (20), où la première partie de conduite (14) entoure la deuxième partie de conduite (16) et est en liaison étanche au fluide des deux côtés du corps de fermeture (20) avec les parties (16', 16") de la deuxième partie de conduite (16).

14. Conduite (10) selon l'une des revendications précédentes, **caractérisée en ce que** le corps de fermeture (20) est constitué d'un ou de plusieurs matériaux élastiques et/ou plastiques.

15. Conduite (10) selon l'une des revendications précédentes, **caractérisée en ce que** le corps de fermeture (20) est élastique, et **en ce que** des moyens sont prévus qui fixent le corps de fermeture (20) dans une position déformée.

16. Procédé d'ouverture d'une conduite selon l'une des revendications 1 à 15, **caractérisé en ce que** le corps de fermeture (20), dans le but de l'ouverture de la conduite (10), est déformé plastiquement à partir de l'état dans lequel celui-ci ferme la conduite (10) de façon qu'un passage à travers la conduite (10) soit libéré.

17. Procédé selon la revendication 16, **caractérisé en ce que** la déformation du corps de fermeture (20) est effectuée au moyen d'un outil (50).

18. Procédé selon la revendication 17, **caractérisé en ce que** dans le cas de l'outil (50), il s'agit d'une pince actionnée manuellement ou du composant d'une machine.

19. Procédé selon la revendication 17 ou 18, **caractérisé en ce que** l'outil (50) présente des mâchoires planes (52).

20. Procédé selon la revendication 17 ou 18, **caractérisé en ce que** l'outil (50) présente une mâchoire (54) avec une saillie (55) et une mâchoire (56) avec un évidement (57) dans lequel s'engage la saillie (55) au moins partiellement lorsque l'outil (50) est fermé.

21. Procédé selon l'une des revendications 16 à 20, **caractérisé en ce que** la déformation du corps de fermeture (20) a lieu de telle sorte que celui-ci, après sa déformation, est fixé dans la conduite (10) à l'encontre d'un déplacement.

22. Tube de prélèvement sanguin ou poche de sang avec une conduite selon l'une des revendications 1 à 15.
